# DEMANDE DE BREVET EUROPEEN

(11) **EP 0 582 514 A1**
(43) Date de publication de la demande: **09.02.1994**
(21) Numéro de dépôt: 93401993.6
(22) Date de dépôt: 02.08.1993
(51) Int. Cl.: A61F 2/38

(54) **Prothèse du genou**

(30) Priorité: 03.08.1992 FR 9209886; 15.06.1993 FR 9307501
(71) Demandeur: IMPLANTS ORTHOPEDIQUES TOUTES APPLICATIONS, S.A.R.L. dite:, F-42000 Saint-Etienne (FR)
(72) Inventeur: Curey, Jean Paul, F-34260 Le Bousquet d'Orb (FR); Richard, Yvonnick, F-56120 Josselin (FR)
(74) Mandataire: Perrier, Jean-Pierre

(57) **Abrégé**

Selon l'invention, les deux condyles (8) de l'élément fémoral (3) présentent, chacun, au moins depuis la zone (E) de cet élément, avec laquelle l'élément rotulien (4) est en contact lorsque la prothèse est en position d'extension maximale, et, au moins jusqu'à la zone (F) de ce même élément, avec laquelle l'élément rotulien (4) est en contact lorsque la prothèse est en position de flexion maximale,
- en section transversale et au moins sur leurs berges internes, un profil externe convexe constant,
- et en section longitudinale, une courbure convexe de rayon (R2) constant et de centre (C2) confondu avec le centre des connexions ligamentaires sur le fémur, tandis que les congés latéraux de l'élément rotulien (4) présentent chacun, en section transversale, une courbure, égale à celle des condyles, et, en section longitudinale un profil concave épousant les berges internes des condyles.

## Description

L'invention concerne une prothèse du genou à glissement composée :
- d'un plateau tibial avec une plate-forme de glissement,
- d'un élément fémoral pourvu de deux condyles qui, identiques et disposés symétriquement et parallèlement de part et d'autre du plan médian vertical de cet élément, sont reliés par un segment intercondylien,
- et d'un élément rotulien, muni de moyens de fixation dans la rotule et de faces de glissement coopérant avec les condyles et présentant, en section longitudinale, un profil concave et, en section transversale, deux congés latéraux.

Les prothèses "à glissement" qui ont succédé aux prothèses "à charnières" n'ont pas supprimé les échecs mécaniques (usure-rupture-descellement-luxation), à court terme et moyen terme, entraînant des ré-interventions.

De nombreuses solutions ont été proposées, toutes basées sur la conception classique d'un genou comportant deux articulations d'architecture et de fonctionnement différents: la principale étant l'articulation fémoro-tibiale "portante", la seconde étant la fémoro-patellaire, ayant un rôle de guidage de l'appareil extenseur.

C'est donc d'abord sur les formes et matériaux des surfaces fémoro-tibiales qu'ont porté l'essentiel des efforts d'amélioration concernant la prothèse du genou. Cependant la relative fréquence des complications fémoro-patellaires déjà citées, coexistant souvent avec des complications de même nature dans le secteur fémoro-tibial, font penser que ces deux articulations doivent travailler en synergie à l'intérieur d'un seul mécanisme répartissant régulièrement et à tout moment les charges transmises aux surfaces de la néo-articulation.

Avec cette théorie mécanique globale de l'articulation du genou prothèsé, diverses tentatives d'amélioration isolée du secteur fémoro-patellaire ont été proposées, mais tous ces essais visaient à renforcer la stabilité de l'implant rotulien, quelle que soit sa forme, à l'intérieur d'une gorge creusée à la face antérieure de l'implant fémoral :
- soit, comme décrit dans U.S. 3 964 106 par le biais de surfaces très congruentes dans le plan sagittal longitudinal ou transversal, le contact "forcé" s'effectuant uniquement dans le creusement fémoral, au centre ou latéralement,
- soit par la recherche d'un alignement de la gorge trochléenne, non plus en fonction de l'axe mécanique global du membre inférieur, mais de l'axe anatomique du fémur pour tenter de neutraliser la tendance à la latéralisation de la rotule, lors de la mobilisation du genou.

L'expérience a démontré les inconvénients de ces méthodes :

Ainsi, le "centrage forcé" de la rotule provoque des contraintes supplémentaires anormales au niveau des surfaces d'ancrage et sur les rotations des segments du membre inférieur l'un par rapport à l'autre.

Inversement, un "décentrage" statique de la gorge de la trochlée de l'implant fémoral pour accompagner une rotule dont la position est nécessairement variable au cours de la flexion-extension, en fonction de la rotation fémoro-tibiale des implants, ne peut représenter qu'une solution théorique au problème de la stabilité de la rotule prothèsée et aucune amélioration par rapport aux prothèses classiques, à trochlée "plates", où la qualité de l'alignement de l'appareil extenseur dépend surtout de l'expérience du chirurgien qui ajuste au mieux la position en rotation relative des composants fémoral et tibial minimisant la déviation en "baïonnette", tout en réglant la tension des formations ligamentaires latérales.

Actuellement, il n'existe aucune garantie de stabilité ni de longévité de l'implant rotulien et, par conséquent, de l'ensemble du genou prothèsé, faute de repères précis dans les trois plans de l'espace où évolue l'articulation du genou. De même, les prothèses de genou actuelles ont une géométrie anormale basée sur l'indépendance des secteurs fémoro-rotulien et fémoro-tibial, qui résulte d'une mauvaise interprétation de l'anatomie du genou normal.

Ainsi, à partir de l'extension, la rotule, qui n'est pas en contact avec le fémur, voit sa course s'allonger et rebondir sur les différents reliefs condyliens qu'elle doit contourner, avec création de chocs. Cela entraîne une réduction des possibilités utiles de flexion et, par l'intermédiaire du tendon rotulien, augmente les contraintes de "cisaillage" sur les surfaces tibiales, elles-mêmes en porte-à-faux alternatif avant-arrière, en raison de la même non concordance des contours condyliens adjacents.

Réciproquement, de la flexion à l'extension, le genou prothèsé subit les mêmes perturbations mécaniques délétères pour l'état des surfaces et les ancrages, tout en exigeant un travail accru du quadriceps élevant fortement la consommation en oxygène, le débit cardiaque et par conséquent la fatigue du patient opéré.

Il est évident que cette mécanique pathologique est directement liée à la géométrie des surfaces de l'implant fémoral dessiné avec des centres et des rayons de courbure multiples et variables, non repérés dans l'espace, et surtout non corrélés avec les centres de la flexion-extension et de rotation du genou normal, genou dont la position des axes, à l'inverse des prothèses existantes, est fixe, connue et correspond aux aires d'insertion des principaux ligaments du genou sur le fémur, réalisant des connexions équidistantes avec la rotule et le tibia.

La présente invention a pour but de remédier aux inconvénients des prothèses existantes, en fournissant une prothèse dont la géométrie générale, plus proche de la physiologie du genou normal, procure un mouvement simple et régulier, aussi bien de l'élément rotulien que de l'élément fémoral, mouvement économisant les surfaces prothétiques et améliorant la longévité des ancrages osseux.

A cet effet, dans la prothèse selon l'invention, les deux condyles de l'élément fémoral présentent, chacun, au moins depuis la zone E de cet élément, avec laquelle l'élément rotulien est en contact lorsque la prothèse est en position d'extension maximale, et, au moins jusqu'à la zone F de ce même élément, avec laquelle l'élément rotulien est en contact lorsque la prothèse est en position de flexion maximale,
- en section transversale et au moins sur leur berge interne, un profil externe convexe constant,
- et en section longitudinale, une courbure convexe de rayon constant et de centre confondu avec le centre des connexions ligamentaires sur le fémur, tandis que les congés latéraux de l'élément rotulien présentent chacun, en section transversale, une courbure égale à celle des condyles, et, en section longitudinale, un profil concave épousant les berges internes de ces condyles.

Grâce à cet agencement, les condyles forment, dans leur partie coopérant avec l'élément rotulien, deux surfaces toriques de section semi-circulaire contre lesquelles s'applique l'élément rotulien par toutes ses surfaces en vis à vis. Il en résulte que le contact entre l'élément rotulien et l'élément fémoral s'effectue par une surface importante, depuis la position d'extension maximale jusqu'à la position de flexion maximale, et que la pression d'application de l'élément rotulien sur l'élément fémoral est considérablement réduite, ce qui supprime tous les risques de luxation, réduit la formation des jeux et augmente la longévité de la prothèse.

Cette géométrie, différente de celles des prothèses connues, est plus proche de la physiologie du genou normal et permet, grâce à son rayon de courbure constant, un mouvement régulier de rotation sphérique autour du composant fémoral, respectivement de l'implant rotulien et de l'élément tibial, qui est très différent de celui des prothèses actuelles faisant alterner des déplacements en rotation avec des déplacements en translation.

Par ailleurs, ce mécanisme sans blocage, par effet de came ou par obstacles au glissement ou au roulement, améliore la fiabilité de la prothèse.

De plus, il augmente l'efficacité et la rentabilité de l'appareil extenseur grâce au bras de levier supplémentaire fourni par le déport avant du centre de gravité de la prothèse par rapport au centre de rotation de la prothèse.

Dans une forme d'exécution, le pont intercondylien de l'élément fémoral est espacé radialement de la face en vis à vis reliant les congés de l'élément rotulien, de manière que seules les berges des condyles guident cet élément rotulien, même lorsqu'il est en vis à vis du pont intercondylien.

Avantageusement, l'élément rotulien est composé :
- d'une partie postérieure comportant sur l'avant, une face plane, orthogonale à l'axe longitudinal de l'élément,
- d'une partie antérieure présentant, sur l'arrière, une face plane, orthogonale à l'axe longitudinal de l'élément,
- et des moyens de liaison de ces deux parties comprenant un tenon, saillant de l'une des deux parties et s'engageant dans une lumière, oblongue et horizontale, ménagée dans l'autre partie, cette lumière assurant la liaison en translation verticale des deux parties et leur liaison en translation horizontale, en tolérant un déplacement relatif de ces deux parties.

Cet agencement procure une liberté de latéralisation et d'autocentrage de la partie osseuse de la rotule par rapport à la prothèse, quel que soit le degré de flexion-extension-rotation axiale du genou et/ou du fémur par rapport au tibia.

Dans une forme d'exécution de l'invention, les moyens assurant la liaison entre les deux parties de l'élément rotulien sont constitués par un tenon axial, saillant vers l'arrière de la partie antérieure, traversant une lumière oblongue de même largeur que son diamètre et ménagée transversalement et dans la partie postérieure de l'élément, et pénétrant dans un canal transversal qui, traversant de part en part cette partie postérieure, constitue réservoir de liquide synovial et cavité d'amortissement des déplacements latéraux relatifs.

Les deux parties de l'élément rotulien étant plaquées l'une sur l'autre par les ligaments rotuliens, lors des mouvements du genou, les déplacements transversaux du tenon dans la lumière oblongue, formant glissière de guidage, déplacent le liquide synovial contenu dans le canal transversal. Par réaction, ce liquide amortit les mouvements relatifs des deux parties. Par ailleurs, le liquide chassé du canal assure une lubrification forcée des surfaces de glissement, à savoir entre les condyles et la partie postérieure de l'élément rotulien et, entre les deux parties de l'élément rotulien.

D'autres caractéristiques et avantages ressortiront de la description qui suit en référence au dessin schématique annexé représentant, à titre d'exemples non limitatifs, plusieurs formes d'exécution de cette prothèse.
Figure 1 est une vue de côté en élévation montrant la position de la prothèse par rapport à l'articulation fémoro-tibiale,
Figure 2 est une vue en perspective montrant les éléments de la prothèse,
Figures 3 et 4 sont des vues en coupe longitudinale de la prothèse montée dans l'articulation et lorsque cette articulation est respectivement en position d'extension maximale et en position de flexion maximale,
Figure 5 est une vue de côté de l'élément fémoral montrant plus en détails ses courbures dans le plan vertical,
Figure 6 est une vue en perspective avec coupe partielle montrant, à échelle agrandie, une forme d'exécution de l'élément rotulien,
Figures 7 et 8 sont des vues en coupe transversale suivant respectivement VII-VII de figure 3 et VII-VII de figure 4,
Figures 9 et 10 sont des vues en perspective avec coupe partielle montrant deux autres formes d'exécution,
Figure 11 est une vue partielle en coupe transversale de l'assemblage des éléments de la prothèse de figure 10.

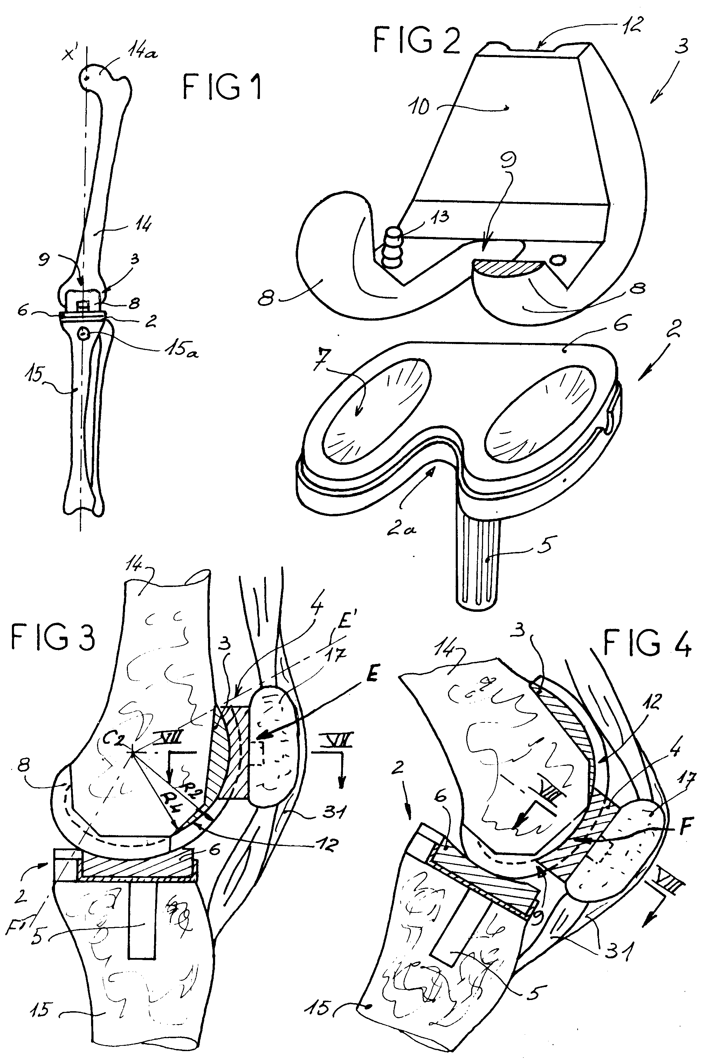

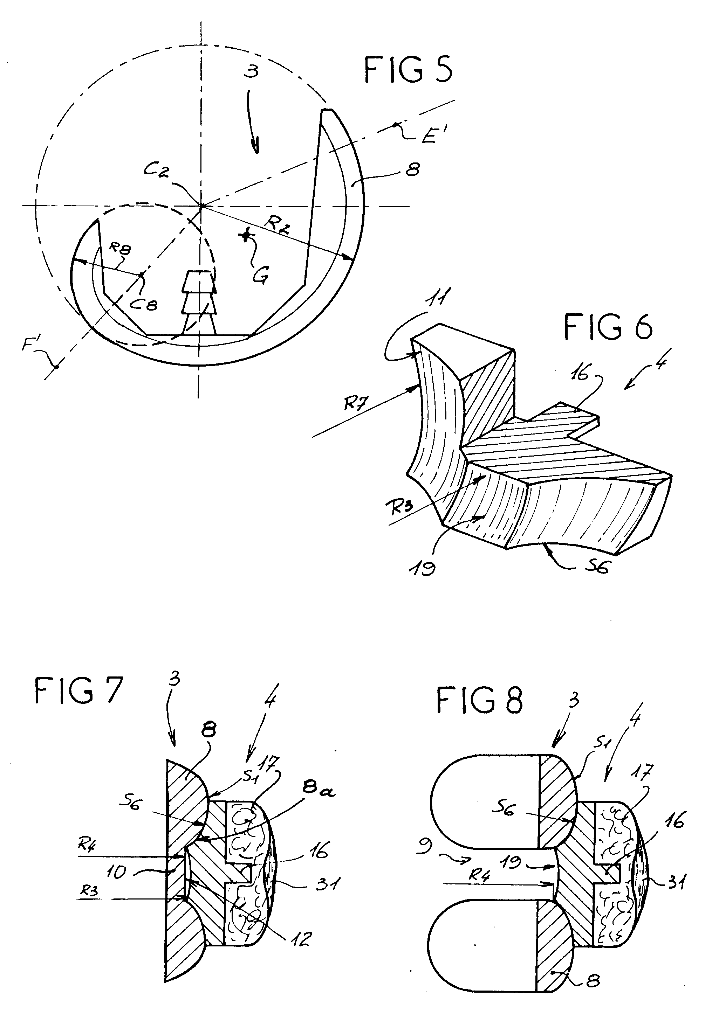

Cette prothèse est composée, de façon connue, d'un plateau tibial désigné de façon générale par la référence 2, d'un élément fémoral 3, et d'un élément rotulien 4, (figures 3 et 4).

Le plateau tibial 2 est solidaire d'une tige 5 se fixant dans le tibia et porte une plate-forme 6, en matériau synthétique et dans laquelle sont ménagées deux cuvettes 7. Il est échancré en 2a pour laisser le libre passage au ligament croisé postérieur.

L'élément fémoral 3 comporte deux condyles 8 séparés par un espace 9 pour le passage des ligaments croisés postérieurs. Les deux condyles 8 sont prolongés sur leur partie antérieure, par un pont intercondylien 10 avec une face externe 12. Chaque condyle 8 est muni d'un tenon 13 assurant son positionnement et contribuant à sa fixation dans le fémur 14.

Cette prothèse est symétrique et peu indifféremment être montée sur une articulation droite ou gauche.

Comme le montre la figure 1, lorsqu'elle est mise en place entre le fémur 14 et le tibia 15, le plan médian longitudinal de la gouttière formant la trochlée contient l'axe mécanique x'-x du membre allant du centre de la tête 14a du fémur 14 au centre de la cheville, en passant par le centre de la tubérosité tibiale 15a.

Aux figures 3 et 4, les traits mixtes E' et F' matérialisent les limites des zones E et F de l'élément fémoral 3, correspondants, celle E, à la zone avec laquelle l'élément rotulien 4 est en contact avec l'élément fémoral lorsque la prothèse est en position d'extension maximale, (figure 3), et, celle F, à la zone avec laquelle le même élément rotulien 4 est en contact avec l'élément fémoral 3 lorsque la prothèse est en position de flexion maximale (figure 4).

Selon l'invention, chaque condyle 8 présente, de la zone E à la zone F, d'une part, en section transversale et comme montré aux figures 5, 7 et 8, un profil externe convexe constant S1 et, d'autre part, en section longitudinale, et comme montré à la figure 5, une courbure convexe de rayon R2 constant et de centre C2. Par ailleurs et comme montré figure 7, le pont intercondylien 10 présente, entre les condyles, une face 12 qui, plane ou concave en section transversale, est convexe en section longitudinale et a un rayon R4, constant et de centre C2, ce rayon ayant une valeur inférieure au plus petit rayon des parties actives des berges internes 8a des condyles 8.

Ce centre C2 est confondu avec le centre des connexions ligamentaires sur le fémur, correspondant lui-même au centre de la flexion-extension du genou normal. C2 est donc en arrière du centre de gravité G de la prothèse.

La figure 5 montre également qu'au-delà de la zone F, et plus précisément sur la limite F' de cette zone, la courbure de rayon R2 et de centre C2 de chaque condyle 8, se raccorde, à une courbure de rayon R8 et de centre C8.

Il s'agit d'un raccordement au sens géométrique du terme, c'est à dire sans formation d'arête pouvant bloquer le roulement des condyles sur le plateau tibial 6. Cette courbure de rayon R8 correspond à la longueur utile du ligament croisé postérieur inséré entre C2 et C8. La différence de valeur entre les rayons R2 et R8 accentue le déplacement de rappel du fémur sur le tibia avec l'aide du ligament croisé postérieur et accentue la flexion tout en tolérant les rotations relatives.

L'élément rotulien 4, montré figure 6, présente, en vis à vis de chaque condyle 8, un congé 11 ayant, transversalement, une forme concave S6 égale à celle S1 des condyles, et, longitudinalement, une forme concave, de rayon R7 et de centre C2, apte à épouser la berge 8a du condyle.

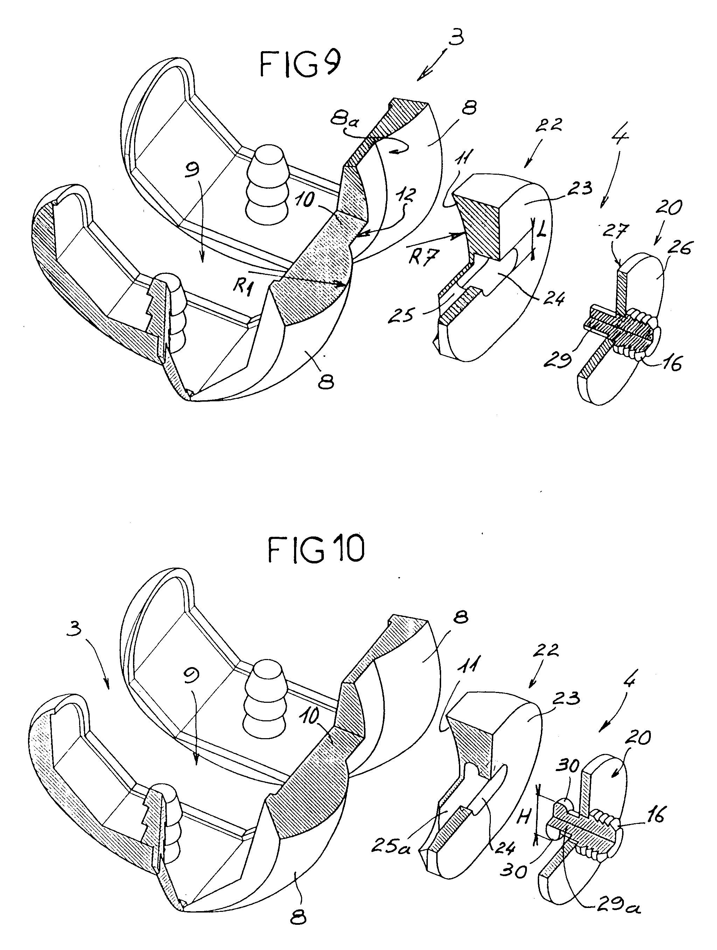

Comme montré figure 6, l'élément rotulien présente également entre les deux congés 11, une face 19 qui, plane ou concave transversalement, est concave longitudinalement suivant un rayon centré en C2 et ayant une valeur constante R3, supérieure à R4, de manière que cette face 19 soit toujours espacée radialement de la face externe 12 du pont intercondylien, comme montré figure 10. Cette disposition favorise le positionnement transversal de l'élément rotulien.

Enfin, l'élément rotulien est muni d'un pion antérieur 16 permettant sa fixation dans la rotule 17.

Cet agencement aménage entre les zones E et F de l'élément fémoral deux surfaces toriques de section semi circulaire, et donne à l'élément rotulien 4 des formes complémentaires parfaitement adaptées pour venir en contact par toute leur surface avec les berges 8a de ces surfaces toriques, comme montré aux figures 7 et 8.

Cette large surface de contact réduit considérablement la pression entre les éléments et, en conséquence, réduit les usures, la formation de jeux fonctionnels, la formation de composantes de réaction sur les autres éléments et les risques de descellement, et augmente la longévité de la prothèse.

Les formes d'exécution représentées aux figures 9 à 11 diffèrent de la précédente par l'élément rotulien 4 qui est structuré pour donner à la rotule 17 une possibilité de latéralisation et d'autocentrage, c'est à dire de déplacement transversal par rapport à la prothèse.

Comme montré plus en détails aux figures 9 et 10, l'élément rotulien 4 est composé d'une partie antérieure 20 et d'une partie postérieure 22. La partie postérieure est réalisée en matériau synthétique biocompatible présentant un excellent coefficient de glissement, et comporte, sur sa partie arrière, les congés 11 épousant les condyles. Sur l'avant, la partie postérieure 22 comporte une face plane 23 de laquelle débouche une lumière oblongue 24 disposée transversalement dans la partie 22, qu'elle traverse de part en part.

La partie antérieure 20 de l'élément rotulien 4 est réalisée en métal et est essentiellement composée d'un disque plan 26, dont la face postérieure 27 est apte à venir en contact de glissement avec la face plane 23 de la partie postérieure 22. Le disque 26 comporte, en saillie sur l'avant, le moyen de liaison avec la rotule 17 et, par exemple, un pion axial 16. Ce pion peut être remplacé par des picots saillants de la face avant du disque 26.

Dans la forme d'exécution représentée à la figure 9, le pion 16 se prolonge sur l'arrière par un tenon axial 29 de dimension diamétrale égale, au jeu fonctionnel près, à la largeur L de la lumière oblongue 24. La longueur de ce tenon 29 lui permet de traverser la lumière oblongue 24 et de pénétrer dans le canal 25.

La forme d'exécution représentée à la figure 10 se différencie de la précédente par le fait que le tenon 29a comporte, à son extrémité libre, deux excroissances 30 lui donnant localement la forme d'un T couché. Ces excroissances ont une largeur égale au diamètre du tenon et forment un bouton de hauteur H, inférieure à la longueur de la lumière oblongue 24, de manière à pouvoir passer à travers celle-ci. Quant au canal 25a, ménagé dans la partie postérieure 22 de l'élément rotulien, il comporte, en section transversale, c'est à dire perpendiculairement à son axe longitudinal, une forme adaptée pour recevoir le tenon et ses excroissances 30.

On notera que, lors de l'assemblage des deux parties de l'élément rotulien, la partie postérieure 22 est pivotée de 90°, de manière que l'axe longitudinal de la lumière 24 soit vertical et coïncide avec le bouton du tenon 29a et que, après que ce bouton ait pénétré dans le canal 25a, il suffit de ramener la partie postérieure 22 dans sa position normale pour assurer le calage en translation de ces deux parties, grâce aux excroissances 30 venant en appui sur les faces internes du canal 25a.

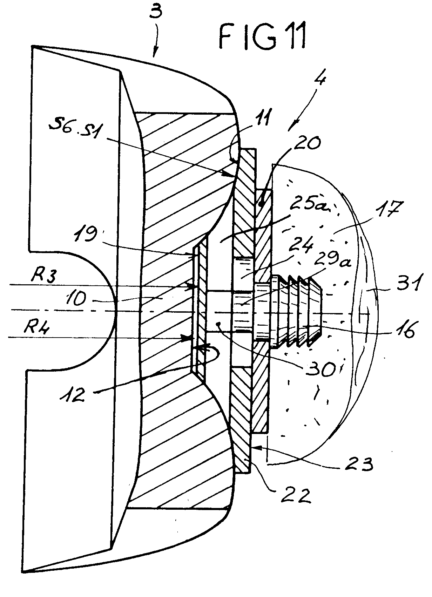

La figure 11 montre que, lorsque les deux parties de l'élément rotulien sont ainsi assemblées, l'élément rotulien est plaqué par les ligaments 31 contre l'élément fémoral 3.

Lors des mouvements du genou, la partie antérieure 20, qui est liée à la rotule 17, peut se déplacer transversalement grâce au jeu ménagé dans la lumière 24 entre le tenon 29 et les extrémités opposées de cette lumière. Par contre, il ne peut pas se déplacer dans le plan vertical, car il est calé par les deux bords parallèles de cette lumière. Il en résulte que la rotule peut se déplacer par rapport à la prothèse, de manière à suivre les mouvements qui lui sont imposés par l'anatomie et le mécanisme de l'articulation, sans que cela affecte le contact de sa partie postérieure avec l'élément fémoral. Cette possibilité de latéralisation de la partie osseuse de la rotule supprime tout risque de transmission de contraintes de torsion par les ligaments rotuliens sur les éléments de la prothèse et toute contrainte parasite pouvant affecter le fonctionnement de cette prothèse.

On notera que le tenon 29 ou 29a, tolère également la rotation de la rotule par rapport à la prothèse.

Un autre avantage de cette construction est que, lors des déplacements de la partie antérieure 20 de l'élément rotulien par rapport à sa partie postérieure 22, les mouvements du tenon 29 ou 29a dans le canal 25 ou 25a se communiquent au liquide synovial contenu dans ce canal et induisent des effets de pression ou de dépression forçant le liquide à circuler entre, d'une part, les profils concaves et convexes, respectivement, des condyles 8 et de la partie postérieure 22 de l'élément rotulien et, d 'autre part, entre les faces planes, respectivement, 23 de la partie postérieure 22 et 27 de la partie antérieure 20 de cet élément rotulien 4. Cela assure un lubrification automatique de la prothèse qui améliore ses conditions de fonctionnement.

Par ailleurs, la pression générée sur le liquide synovial assure, par réaction, l'amortissement du déplacement du tenon 29-29a, et, en conséquence, l'amortissement des mouvements relatifs de la rotule 17 par rapport aux autres éléments de la prothèse.

## Revendications

1. Prothèse du genou à glissement, du type composé :
- d'un plateau tibial (2) avec une plate-forme de glissement (6),
- d'un élément fémoral (3) pourvu de deux condyles (8) qui, identiques et disposés symétriquement et parallèlement de part et d'autre du plan médian vertical de cet élément, sont reliés par un segment intercondylien antérieur (10),
- et d'un élément rotulien (4), muni de moyens (16) de fixation dans la rotule (15) et de congés latéraux (11) coopérant avec les condyles (8) et présentant, un profil concave tant en section transversale, qu'en section longitudinale, **caractérisée en ce que** les deux condyles (8) de l'élément fémoral (3) présentent, chacun, au moins depuis la zone (E) de cet élément, avec laquelle l'élément rotulien (4) est en contact lorsque la prothèse est en position d'extension maximale, et, au moins jusqu'à la zone (F) de ce même élément, avec laquelle l'élément rotulien (4) est en contact lorsque la prothèse est en position de flexion maximale,
- en section transversale et au moins sur leurs berges internes (8a), un profil externe convexe constant (S1),
- et en section longitudinale, une courbure convexe de rayon (R2) constant et de centre (C2) confondu avec le centre des connexions ligamentaires sur le fémur, tandis que les congés latéraux (11) de l'élément rotulien (4) présentent chacun, en section transversale, une courbure (S6), égale à celle (S1) des condyles, et, en section longitudinale un profil concave épousant les berges internes (8a) des condyles.

2. Prothèse selon la revendication 1, **caractérisée en ce que** la face antérieure (12) du pont intercondylien (10) de l'élément fémoral (3) est espacé radialement de la face en vis à vis (19) reliant les congés de l'élément rotulien (4), de manière que seules les berges (8a) des condyles (8) guident cet élément rotulien (4), même lorsqu'il est en vis à vis du pont intercondylien (10).

3. Prothèse selon l'une quelconque des revendications 1 et 2, **caractérisée en ce que** la face (19) de l'élément rotulien (4) est concave dans le plan transversal.

4. Prothèse selon l'une quelconque des revendications 1 et 3, **caractérisée en ce que** la courbure convexe de rayon (R2) de chaque condyle (8) se raccorde, en limite de la zone (F), à une courbure convexe de rayon (R8) plus petit et de centre (C8).

5. Prothèse selon l'une quelconque des revendications 1 à 4, **caractérisée en ce que** l'élément rotulien (4) est composé :
- d'une partie postérieure (22) comportant sur l'avant, une face plane (23), orthogonale à l'axe longitudinal de l'élément,
- d'une partie antérieure (20) présentant, sur l'arrière, une face plane (27), orthogonale à l'axe longitudinal de l'élément,
- et des moyens de liaison de ces deux parties (20-22) comprenant un tenon (29), saillant de l'une des deux parties et s'engageant dans une lumière (24), oblongue et horizontale, ménagée dans l'autre partie, cette lumière assurant la liaison en translation verticale des deux parties et leur liaison en translation horizontale, en tolérant un déplacement relatif de ces deux parties.

6. Prothèse selon la revendication 5, **caractérisée en ce que** les moyens assurant la liaison entre les deux parties (20-22) de l'élément rotulien (4) sont constitués par un tenon axial (29a), saillant vers l'arrière de la partie antérieure (30), traversant une lumière oblongue (24) de même largeur que le diamètre du tenon (29) et ménagée transversalement dans la partie postérieure (20), ce tenon pénétrant dans un canal transversal (25-25a) qui, traversant de part en part cette partie postérieure (20), constitue réservoir de liquide synovial et cavité d'amortissement des déplacements latéraux relatifs.

7. Prothèse selon la revendication 6, **caractérisée en ce que** le tenon (29a) est muni, à son extrémité libre arrière de deux excroissances opposées (30), disposées dans le plan vertical et lui donnant localement une forme en "T" couché, dont la barre supérieure est apte à passer à travers la lumière oblongue (24) de la partie postérieure (20), alors pivotée de 90°, tandis que le canal transversal (25a) de la partie postérieure (20) présente, en section transversale verticale, des formes et dimensions identiques, au jeu fonctionnel près, à celles de l'extrémité du tenon (29).

8. Prothèse selon l'une quelconque des revendications 5 à 7, **caractérisée en ce que** la partie postérieure (20) de l'élément rotulien (4) est en matériau synthétique biocompatible et à fort coefficient de glissement, tandis que sa partie antérieure (22) est en métal.
